(19) **Europäisches Patentamt**

**European Patent Office**

**Office européen des brevets**

(11) **EP 0 692 986 B1**

(12) **FASCICULE DE BREVET EUROPEEN**

(45) Date de publication et mention
de la délivrance du brevet:
**19.07.2000 Bulletin 2000/29**

(21) Numéro de dépôt: **95908998.8**

(22) Date de dépôt: **08.02.1995**

(51) Int. Cl.⁷: **A61L 27/00**

(86) Numéro de dépôt international:
**PCT/FR95/00150**

(87) Numéro de publication internationale:
**WO 95/21634 (17.08.1995 Gazette 1995/35)**

(54) **COMPOSITION POUR BIO-MATERIAU; PROCEDE DE PREPARATION**

BIOMATERIALSZUSAMMENSETZUNG UND VERFAHREN ZU SEINER HERSTELLUNG

BIOMATERIAL COMPOSITION AND METHOD FOR PREPARING THE SAME

(84) Etats contractants désignés:
**AT BE CH DE DK ES FR GB GR IE IT LI LU MC NL PT SE**

(30) Priorité: **08.02.1994 FR 9401414**

(43) Date de publication de la demande:
**24.01.1996 Bulletin 1996/04**

(73) Titulaire:
**CENTRE NATIONAL DE
LA RECHERCHE SCIENTIFIQUE (CNRS)
75016 Paris (FR)**

(72) Inventeurs:
• **DACULSI, Guy**
**F-44360 Vigneux-de-Bretagne (FR)**
• **WEISS, Pierre**
**F-44300 Nantes (FR)**
• **DELECRIN, Joel**
**F-44000 Nantes (FR)**

• **GRIMANDI, Gael**
**F-44000 Nantes (FR)**
• **PASSUTI, Norbert**
**F-44230 Saint-Sebastien-sur-Loire (FR)**
• **GUERIN, François**
**F-78460 Chevreuse (FR)**

(74) Mandataire: **Le Guen, Gérard et al
CABINET LAVOIX
2, place d'Estienne d'Orves
75441 Paris Cédex 09 (FR)**

(56) Documents cités:
**EP-A- 0 115 549          EP-A- 0 511 868
WO-A-86/01113          GB-A- 2 248 232**

• **PATENT ABSTRACTS OF JAPAN vol. 15, no. 128
(C-0818) 28 Mars 1991 & JP,A,03 011 006
(SANKIN KOGYO KK) 18 Janvier 1991 cité dans
la demande**

## Description

L'invention concerne une composition injectable d'un bio-matériau de comblement de tissus de soutien, dentaires, osseux et ostéo-articulaires destiné à générer une fonction de résorption/substitution.

De l'état de la technique sont connus des substituts osseux à base de particules de phosphate de calcium et d'une colle biologique.

Ainsi, G. DACULSI et al. ont décrit dans Ann. Oto. Rhino. Laryngol. 101:1992, l'efficacité d'une composition microporeuse biphasique de phosphate de calcium pour l'oblitération de la cavité mastoïdienne.

Les mêmes auteurs ont également rapporté l'efficacité d'une composition macroporeuse de phosphate de calcium biphasique pour la réparation chirurgicale d'os longs (Journal of Biomedical Materials Research, Vol. 24, 379-396) et dans les arthrodèses vertébrales (Clinical Orthopadics and Related Research, 248, 1989, 169-175).

D'autre part, JP 3 011 006 décrit un cément pour tissus durs comprenant une phase minérale constituée d'au moins 60% de phosphate tri-calcique alpha et de l'hydroxyapatite et/ou un mono-phosphate de calcium ; et une phase liquide comprenant de la carboxyméthylcellulose.

Une telle composition présente cependant l'inconvénient, en raison de la solubilité trop grande du phosphate tri-calcique $\alpha$, de ne pas être suffisamment stable pour permettre un processus de résorption/substitution du tissu dur. En outre, une telle composition est susceptible de générer des processus inflammatoires néfastes. En outre, ce mélange constitue un ionomère de calcium impropre à l'injection après quelques minutes par durcissement du mélange dès sa constitution. Cette association présente une double instabilité, une contraction volumétrique avec relargage d'eau après plusieurs jours, et surtout une chute de la viscosité après stérilisation à l'autoclave du mélange. Elle ne permet pas la réalisation d'un matériau "prêt à l'emploi", stérile, injectable.

La présente invention s'est fixé pour but de fournir une composition de bio-matériau chargée en phase minérale, réhabitable, et injectable par voie percutanée.

En particulier, ce bio-matériau doit présenter les propriétés suivantes :

- Il doit être stérilisable.
- Il ne doit pas être toxique in vivo.
- Il doit posséder une forte charge minérale induisant un front de minéralisation.
- L'agent de dispersion ne doit avoir qu'un rôle de vecteur et doit disparaître dans le temps sans réaction inflammatoire nocive.
- Sa rhéologie doit être telle qu'elle permette l'injection.
- Il doit être facile d'emploi.

Ce but a été atteint par la présente invention, dont l'objet est une composition pour bio-matériau de résorption/substitution de tissus de soutien, dentaires, osseux et ostéo-articulaires composée de :

- 40 à 75% en poids d'une phase minérale comprenant soit un mélange de phosphate tricalcique $\beta$ (A) et d'hydroxyapatite (B), dans un rapport A/B compris entre 20/80 et 70/30, soit du calcium-titane-phosphate ($Ca(Ti)_4(PO_4)_6$) (C), et
- 60 à 25% en poids d'une phase liquide comprenant une solution aqueuse d'un polymère dérivé de la cellulose.

Le calcium-titane-phosphate (CTP) de formule $Ca(Ti)_4(PO_4)_6$ est de préférence du type Calci-Métallo phosphate Nasicon-like.

Avantageusement, la phase minérale comprend 40% de composé (A) et 60% d'hydroxyapatite (B).

Elle consiste en un fritté haute température, broyé et calibré en poudre ou en granulés dont les particules ont un diamètre de 80 $\mu$m à 200 $\mu$m lors de l'élaboration de la composition. Le choix du diamètre particulaire est guidé par la cinétique de résorption d'une part et la rhéologie à l'injection d'autre part. Des particules de diamètre inférieur à 80 $\mu$m ont une cinétique de résorption trop rapide et celles de diamètre supérieur à 200 $\mu$m présentent des problèmes de rhéologie à l'injection.

Le polymère viscoélastique de la phase liquide est un polymère non ionique, notamment de l'hydroxypropylméthylcellulose. Une hydroxypropylméthylcellulose préférée présente une substitution molaire par des groupements méthyles de 19 à 24% et par des groupements hydroxypropyles de 4 à 12%. Elle présente un fort degré de polymérisation. De préférence, le poids moléculaire moyen en poids est supérieur à 100 000 et avantageusement compris entre 500 000 et 1 000 000.

La phase aqueuse est déterminante eu égard aux propriétés rhéologiques et donc à la visco-élasticité de la composition finale destinée à être injectée.

A cet effet, la concentration en polymère est avantageusement comprise entre 0,5 et 4%, de préférence 0,5 et 2% en poids par rapport au poids de la phase aqueuse.

La composition selon l'invention est obtenue par mélange des constituants de la phase minérale et de la phase aqueuse.

Les granulés ou la poudre de phosphate tricalcique $\beta$ ou de CTP et d'hydroxyapatite constituant la phase minérale sont obtenus comme décrit par DACULSI et al. (Rev. Chir. Orthop., 1989, 75(2) : 65-71).

Une stérilisation par oxyde d'éthylène n'est pas possible pour un matériau "prêt à l'emploi". Il faut en effet stériliser sous leur forme sèche les composants du mélange ce qui impose une manipulation pour le chirurgien avant l'injection. Cette manipulation est difficile et non reproductible.

Selon l'invention, la stérilisation de la composition est réalisée par dissolution du constituant polymère de la phase aqueuse dans de l'eau à une viscosité déterminée en fonction de la viscosité finale souhaitée. La solution obtenue est ensuite mélangée avec la phase minérale et la composition résultante introduite dans des flacons de conditionnements scellés et stérilisés à l'autoclave à 121°C pendant 20 minutes.

La viscosité initiale de la composition (concentration en polymère) doit être adaptée pour obtenir la viscosité désirée après stérilisation, c'est-à-dire la concentration en polymère telle que définie ci-dessus.

La composition selon l'invention peut être utilisée comme matériau de comblement osseux de tissus durs de l'organisme, destiné à générer une fonction de résorption/substitution. Elle peut notamment être utilisée comme matériau de comblement associé à des implants ou des prothèses articulaires ou comme matériau de comblement lors de résections tumorales.

Un exemple d'application est le remplacement de disques intervertébraux.

La composition peut être injectée par voie percutanée.

L'injection peut être réalisée à l'aide d'un système comportant une seringue stérilisable et des embouts munis de pistons à usage unique, par exemple le système commercialisé par HAWE NEOS DENTAL, comprenant une seringue stérilisée à l'autoclave (Réf. N° 440, Seringue Hawe-Centrix C-R [R], Mark III) et des embouts (Réf. N° 445).

On donnera ci-après les résultats obtenus chez l'animal avec un exemple de composition selon l'invention.

## Exemple de composition

On a mélangé des granulés composés de 40% en poids de phosphate tricalcique β et 60% d'hydroxyapatite, dont 95% avaient un diamètre particulaire compris entre 80 et 200 μm, mélangé à une solution aqueuse contenant 2% d'hydroxypropylméthylcellulose qui présente une substitution molaire par des groupements méthyles de 19 à 24% et hydroxypropyles de 4 à 12% ainsi qu'un fort degré de polymérisation, de façon à obtenir une composition comprenant 57,5% en poids de phosphate tricalcique β et d'hydroxyapatite.

La composition ainsi préparée a été stérilisée à l'autoclave.

## Etude in vivo chez le chien Beagle

## A/ Protocole expérimental

Les deux voies d'abord successives décrites par R.K. GURR et P. Mc AFEE ("Roentgenographic and Biomechanical analysis of lumbar fusions : A canine model" ; J.Orthop.Res., 1989, 7:838-848) ont été reprises sans toutefois entreprendre un geste à canal rachidien ouvert ; par contre, une déstabilisation a été réalisée sur deux étages en L3/L4 et L4/L5.

La résection a été limitée aux ligaments supra et interépineux entre L3 et L5. La facectomie a compris les processus articulaires (PA) caudaux de L3, les PA crâniaux et caudaux de L4 et enfin, les PA crâniaux de L5.

Au niveau de la colonne antérieure, une résection discale a été pratiquée en L3/L4 et en L4/L5 après section de LLA et fenestration discale.

Le chien a été installé en décubitus ventral, les 4 pattes étant attachées. Les processus épineux (PE) lombaires de L2, L3, L3, L4, L5 et L6 ont été repérés par rapport à la 13ème côte, et la peau et le fascia thoracolombaire le long de la ligne médiane des épineuses incisés.

La masse musculaire commune a été dégagée en sous-périosté à la rugine de chaque côté, et a permis ainsi la dissection des lames jusqu'aux processus articulaires.

L'hémostase a été assurée par tamponnement et coagulation si besoin. Ensuite, un contrôle a été réalisé pour repérer de façon certaine les étages L3, L4 et L5, et la dissection de la base des processus transversaires (PT) y a été poursuivie.

Ceci a permis le repérage du point de visée pédiculaire : il se situait en dehors du massif articulaire sur une ligne horizontale passant par la moitié inférieure de la base du PT, la visée se faisant à 45° de dehors en dedans et d'arrière en avant, dans un plan strictement frontal.

La voie d'abord antérieure du rachis lombaire a été réalisée dans le même temps opératoire, du côté gauche, afin de minimiser les risques vasculaires avec la veine cave.

L'animal a été installé en décubitus latéral droit, avec un billot au niveau de la charnière thoracolombaire afin de bien dégager l'angle sous-costal gauche.

Quelques dissections sur cadavres de chien Beagle ont permis de constater que l'abord direct sous la 13ème côte permettait d'aborder assez facilement les étages discaux L3/L4 et L4/L5.

L'incision cutanée a été tracée sur une ligne parallèle au bord inférieur de la 13ème côte, 3 à 4 cm environ en dessous de celle-ci.

Elle débutait à un bon travers de main de l'incision postérieure, afin d'éviter tout risque de nécrose cutanée, et s'étendait sur une dizaine de centimètres.

Les muscles obliques ont été dissociés dans le sens de leurs fibres ; le respect du muscle tranverse a permis le décollement rétro-péritonéal sans risque de pneumo-péritoine, car chez le chien, le péritoine adhère fortement à la face profonde du muscle tranverse et se déchire facilement.

Ce même muscle a été désinséré au niveau de ses attaches rachidiennes, et a découvert les muscles psoas et carré des lombes masquant la face antérieure du rachis.

L'accès au corps vertébraux est barré par les artères et veines lombaires gauches qui ont été liées et sectionnées, ainsi qu'un nerf splanchnique lombaire.

Le plan musculaire antérieur a été libéré au bistouri électrique en H, en réalisant deux lambeaux musculaires pédiculés, puis la face antérieure des disques a été dégagée à la spatule et à la pince gouge. L'aorte et le péritoine ont été réclinés et protégés par une valve souple sous le corps vertébral.

Après un nouveau repérage par amplificateur de brillance, l'excision discale a été réalisée : dans un premier temps, une fenêtre discale a été taillée avec le bistouri poignard, et par cette ouverture, le nucleus a été progressivement évidé par des curettes de taille progressive.

L'ablation s'est poursuivie jusqu'aux plateaux vertébraux qui ont été avivés, pour être en zone de tissu spongieux.

Le remplissage du vide discal aux deux étages a été obtenu par le système d'injection décrit cidessus. Le lambeau musculaire a été repositionné à la face antérieure du rachis par quelques points, et la paroi a été refermée en trois plans, sans drainage.

Le matériau multiphasé a été injecté sous pression dans le vide discal en L3/L4 et L4/L5, après ostéosynthèse.

Les animaux ont été sacrifiés à 6 mois par injection léthale de Nesdonal [R] .

**B/ Résultats**

Sur un lot de 8 chiens, deux complications neurologiques immédiates à type de paraplégie par atteinte du motoneurone central se sont produites. Les animaux ont été sacrifiés à J6.

Les 6 autres chiens n'ont posé aucun problème : dès la 24ème heure, ils ont repris la marche, et dès la fin de la première semaine post-opératoire, la course et les jeux.

Ces 6 chiens ont été sacrifiés 6 mois après l'injection.

Sur des coupes histologiques réalisées à 2 mois et demi, on a observé un néo-tissu osseux pratiquement mature : en effet, les ponts osseux inter-particulaires recréent une trame osseuse à mailles serrées et l'os apparait nettement de structure lamellaire.

Pratiquement, tout le bio-matériau initial a été remplacé par cette architecture, ce qui signifie une bonne diffusion cellulaire à l'intérieur du produit sans réaction inflammatoire.

Ces bons résultats ont été confirmés à 6 mois, où le néo-tissu osseux avait poursuivi sa maturation.

Des études complémentaires de biocompatibilité (injections en site sous-cutané, musculaire et cartilagineux) ont en outre montré une bonne tolérance et une dégradation rapide du matériau injecté.

**<u>Etude in vivo chez la lapine White New Zealand</u>**

**A/ Protocole expérimental**

Les animaux ayant participé à l'étude étaient tous matures et de poids homogène (2,8 kg environ). Le site d'injection retenu était le site endo-médullaire fémoral.

Chaque animal a été opéré des deux fémurs, mais à des dates différentes (délai minimum d'une semaine entre chaque intervention), car le lapin supporte mal la déperdition sanguine d'une intervention bilatérale. Le fémur gauche a toujours été opéré le premier.

Le membre opéré a été tondu quelques minutes avant l'intervention, dans une pièce close éloignée de la salle d'intervention. Il n'a pas été utilisé d'anti-bioprophylaxie.

Le protocole anesthésique a été le suivant :

- 15 minutes avant l'intervention, injection intra-musculaire de 250 mg de chlorhydrate de kétamine (Kétalar [R] , Parke Davis, Courbevoie).
- 1 à 2 minutes avant l'intervention, anesthésie locale et intra-articulaire du genou, à l'aide d'un mélange de chlorhydrate de lidocaïne à 1% (Xylocaïne 1% [R] , Astra France, Nanterre) et de chlorhydrate de lidocaïne à 1% + épinéphrine à 1/100 000 (Xylocaïne adrénaline [R] , Astra France, Nanterre) dans des proportions respectives de 2/3 - 1/3 ;
- compensation per-opératoire des pertes sanguines par 15 ml de Ringer Lactate et Glucose 5% [R] (Labo. Aguettant, Lyon), injecté en 3 fois par une voie d'abord intra-veineuse auriculaire.

L'intervention a été effectuée dans des conditions d'aseptie stricte.

L'animal anesthésié est placé en décubitus dorsal, les 4 membres attachés. Un billot est glissé sous le genou du côté opéré pour maintenir une flexion d'environ 30° de ce genou. Le site opératoire est alors désinfecté par une solution de polyvidone iodée avant et après l'anesthésie locale et intra-articulaire.

Le site opératoire est ensuite isolé par un champ stérile percé.

Les étapes sont les suivantes :

- incision cutanée para-patellaire interne d'environ 3 cm de long ;
- coagulation à la pince d'un pédicule vasculaire constant situé à la partie supérieure de l'incision ;

- incision de l'aileron patellaire interne et de la capsule articulaire ;
- luxation latérale de la patella ;
- forage, à l'aide d'une mèche de 2 mm de diamètre, d'un canal dans la gorge trochléenne, en se laissant "guider" par l'anatomie endo-médullaire du fémur ;
- alésage prudent de la cavité endo-médullaire à l'aide d'une mèche de 4 mm de diamètre, en se guidant sur le forage précédent ;
- lavage abondant, au sérum physiologique, de la cavité médullaire fémorale, afin d'évacuer en totalité la graisse endo-médullaire et les débris osseux de l'alésage ;
- injection lente du matériau (décrit ci-dessus), de façon rétrograde, dans la cavité médullaire fémorale alésée ;
- introduction manuelle de l'implant (broche en alliage de titane ($Ti_6Al_4V$) à surface lisse de 2,5 cm de diamètre) dans sa totalité afin qu'il ne fasse pas saillie dans l'articulation ;
- nettoyage au sérum physiologique et à la spatule, du surplus de matériau évacué lors de la mise en place de l'implant ;
- reposition rotulienne ;
- fermeture, en un seul plan, de la capsule et de l'aileron patellaire par un surjet étanche de Vicryl [R] Déc. 2 ;
- fermeture cutanée par un surjet étanche de Vicryl [R] Déc. 2 ;
- pulvérisation cutanée, sur la voie d'abord, d'un film contenant de l'auréomycine.

Durée de l'intervention : environ 25 minutes.

Aucune antibiothérapie n'a été utilisée en per-opératoire ni en post-opératoire.

Tous les animaux ont été sacrifiés par une injection léthale intraveineuse de 0,25 gramme de thiopental sodique (Nesdonal [R], Spécia Rhône-Poulenc Paris).

**B/ Résultats**

1. Analyse histologique (12 lapins)

Des coupes histologiques réalisées à 1, 3, 6 et 9 semaines ont montré une progression centripète de l'apposition osseuse avec colonisation progressive des particules de la phase minérale, puis apparition d'os lamellaire en même temps que disparaissaient les particules constituant la phase minérale, particulièrement rapide en site épiphysaire.

A 12 semaines, les particules du site épiphysaire et des sites métaphysaire et diaphysaire étaient fragmentés dans une proportion importante et colonisés par une structure osseuse lamellaire à maille serrée, organisée en grande partie selon le mode haversien.

L'apposition osseuse au contact de l'implant était de 43,7 ± 5,2. La surface occupée par les particules de la phase minérale était de 28,6 ± 13 2%. On n'observe pas de tissu fibreux.

Une étude comparative avec le polyméthacrylate de méthyle au lieu de la composition selon l'invention a montré l'apparition d'un réseau fibrillaire progressivement colonisé par une substance ostéoïde non minéralisée.

2. Tests mécaniques d'extraction

L'objectif de ces tests a été d'évaluer la force nécessaire à l'arrachement des implants hors du fût fémoral, à différents délais.

Les fémurs congelés ont été coupés sur machine Isomet Plus Precision Saw [R], transversalement, juste au-dessus de l'extrémité supérieure de la broche.

Le système d'ancrage est composé :

- d'une cupule en aluminium, dans laquelle l'épiphyse distale du fémur est scellée à l'aide d'une dose de ciment acrylique. Grâce à un dispositif d'autocentrage s'adaptant sur la cupule et sur la partie externe de l'implant, le fémur est scellé de façon à ce que l'implant soit parallèle à l'axe de traction ;
- d'un cardan, vissé au centre de la partie inférieure de la cupule, et assurant une orientation possible dans les trois plans de l'espace.

L'ensemble cupule-cardan a été solidarisé au plateau d'une machine MTS 810 [R] (Material Testing System, USA).

Le système de traction est composé :

- d'un mandrin à crémaillère Black et Decker [R] qui enserre l'extrémité proximale de la broche ;
- d'un câble, reliant le mandrin à la jauge de contrainte, cette dernière étant fixée sur la partie mobile de la machine MTS 810 [R].

Réalisation pratique :

La vitesse de déplacement de la cellule de traction a été fixée à 0,5 mm par minute.

L'enregistrement des tests a été fait sur une table traçante. Pour chaque échantillon, une courbe typique force (Newton) - déplacement (mm) a été obtenue.

La résistance maximale à l'extraction (Re en MPa) a été déterminée par la formule : $Re = F/\pi DL$ ,

- F étant la force maximale appliquée à l'implant pendant le test d'extraction (en Newtons),
- D étant le diamètre de l'implant (2,5 mm),
- L étant la longueur de l'implant en contact avec l'os (30 mm).

L'analyse statistique des résultats a été pratiquée selon le test ANOVA d'analyse de variance avec un risque α = 0,05.

Les tests ont été réalisés sur 36 fémurs. A 12 semaines, la résistance à l'extraction est en moyenne de 58,83 (± 17 83) MPa pour la composition selon l'invention.

Par comparaison, pour le ciment à base de méthacrylate de méthyle, la résistance moyenne était de 140,33 (± 8,96) MPa.

**Revendications**

1.    Composition pour bio-matériau de résorption/substitution de tissus de soutien, dentaires, osseux et ostéo-articulaires composée de :

   -    40 à 75% en poids d'une phase minérale comprenant soit un mélange de phosphate tricalcique β (A) et d'hydroxyapatite (B), dans un rapport A/B compris entre 20/80 et 70/30, soit du calcium-titane-phosphate (Ca(Ti)$_4$ (PO$_4$)$_6$) (C), et
   -    60 à 25% en poids d'une phase liquide comprenant une solution aqueuse d'un polymère dérivé de la cellulose.

2.    Composition selon la revendication 1, caractérisée en ce que la phase minérale comprend 40% du composé (A) et 60% d'hydroxyapatite (B).

3.    Composition selon la revendication 1 ou 2, caractérisée en ce que la phase minérale est constituée d'une poudre de granulométrie comprise entre 80 à 200 μm de diamètre.

4.    Composition selon la revendication 1 ou 2, caractérisée en ce que la phase liquide comprend de l'hydroxypropylméthylcellulose.

5.    Composition selon l'une quelconque des revendications précédentes, caractérisée en ce que la concentration du polymère dans la phase liquide est de 0,5 à 4% en poids, de préférence 0,5 à 2% en poids.

6.    Procédé de préparation d'une composition selon l'une quelconque des revendications précédentes, caractérisé en ce que :

   -    l'on mélange la phase minérale et le polymère de la phase liquide dans une solution aqueuse de viscosité déterminée, et l'on stérilise la composition liquide ainsi obtenue.

**Claims**

1.    Composition for a biomaterial for absorption/substitution of support, dental, osseous and osteo-articular tissues which comprises:

   -    40 to 75% by weight of a mineral phase which comprises either a mixture of β-tricalcium phosphate (A) and hydroxy apatite (B), in a ratio A:B of between 20:80 and 70:30, or calcium-titanium-phosphate (Ca(Ti)$_4$ (PO$_4$)$_6$) (C), and
   -    60 to 25% by weight of a liquid phase which comprises an aqueous solution of a polymer derived from cellulose.

2.    Composition according to claim 1, characterised in that the mineral phase comprises 40% compound (A) and 60% hydroxyapatite (B).

3.    Composition according to claim 1 or 2, characterised in that the mineral phase is constituted by a powder having grains measuring from 80 to 200 μm in diameter.

4.    Composition according to claim 1 or 2, characterised in that the liquid phase comprises hydroxypropylmethylcellulose.

5.    Composition according to any one of the preceding claims, characterised in that the polymer concentration in the liquid phase is from 0.5 to 4% by weight, preferably from 0.5 to 2% by weight.

6.    Process for preparing a composition according to any one of the preceding claims, characterised in that:

   -    the mineral phase and the polymer of the liquid phase in an aqueous solution having a specific viscosity are mixed and the liquid composition so obtained is sterilised.

**Patentansprüche**

1.    Bio-Materialzubereitung zur Resorption/Substitution von Stützgewebe, Zahngewebe, Knochengewebe und Knochengelenkgewebe, gebildet aus:

   -    40 bis 75 Gew.-% einer mineralischen Phase, die entweder eine Mischung aus β-Tricalciumphosphat (A) und Hydroxyapatit (B) in einem Verhältnis A/B zwischen 20/80 und 70/30 oder Calcium-Titan-Phosphat (Ca(Ti) (PO$_4$)$_6$) (C) umfaßt, und
   -    60 bis 25 Gew.-% einer flüssigen Phase umfassend eine wäßrige Lösung eines von Cellulose abgeleiteten Polymers.

2.    Zubereitung nach Anspruch 1, dadurch gekennzeichnet, daß die mineralische Phase 40 % der Verbindung (A) und 60 % Hydroxyapatit (B) umfaßt.

3. Zubereitung nach Anspruch 1 oder 2, dadurch gekennzeichnet, daß die mineralische Phase aus einem Pulver mit einer Teilchengröße mit einem Durchmesser zwischen 80 bis 200 μm gebildet ist.

4. Zubereitung nach Anspruch 1 oder 2, dadurch gekennzeichnet, daß die flüssige Phase Hydroxypropylmethylcellulose umfaßt.

5. Zubereitung nach irgendeinem der vorhergehenden Ansprüche, dadurch gekennzeichnet, daß die Konzentration des Polymeren in der flüssigen Phase 0,5 bis 4 Gew.-%, vorzugsweise 0,5 bis 2 Gew.-%, beträgt.

6. Verfahren zur Herstellung einer Zubereitung nach einem der vorhergehenden Ansprüche, dadurch gekennzeichnet, daß man:

   - die mineralische Phase und das Polymer der flüssigen Phase in einer wäßrigen Lösung vorbestimmter Viskosität vermischt und die in dieser Weise erhaltene flüssige Zubereitung sterilisiert.